# EUROPEAN PATENT APPLICATION

(11) **EP 0 866 327 A2**
(43) Date of publication of application: **23.09.1998**
(21) Application number: 98302145.2
(22) Date of filing: 23.03.1998
(51) Int. Cl.: G01N 3/08, G01N 33/44, G01B 11/30

(54) **Testing apparatus**

(30) Priority: 21.03.1997 GB 9705946
(71) Applicant: The University of Hull, Kingston-upon-Hull HU6 7RX (GB)
(72) Inventor: Taylor, Paul Michael, Goxhill, North Lincs. (GB); Mendes, Jose de Araujo, 4730 Vila Verde (PT)
(74) Representative: Knott, Stephen Gilbert

(57) **Abstract**

A testing apparatus which is particularly suitable for leather comprises two pairs of rollers between which the leather passes. By adjusting the speeds and spacings of the rollers and by measuring the torques and positions of the rollers, various characteristics of the leather can be determined. For example, the Young's Modulus can be determined to find low strain regions, a measure of softness can be obtained, shear stress across the thickness can be determined, as can compressiblity and thickness. A laser sensor can be provided to measure surface profile (roughness).

## Description

The invention relates to testing apparatus and in particular to testing apparatus for elastic materials.

An example of an elastic material is leather. Leather is an expensive component of many products such as shoes, furniture, car seats, garments and bags. Since it is a natural material, a tanned leather hide will contain visual and hidden flaws. In addition, its mechanical properties vary over the hide so that certain pieces must be cut from certain parts of the skin and in certain orientations to make the product easy to manufacture and comfortable to wear or use.

At present, hides are tested by visual and tactile inspection by skilled operatives at tanneries and at places of manufacture, with faulty regions being cut out or avoided. Mechanical testing requires the removal of samples and thus gives information only about the sample and not about the rest of the hide. Further, some current testing is destructive. For this reason, testing gives little useful information.

According to a first aspect of the invention, there is provided apparatus for testing a piece of elastic material such as leather comprising first and second pairs of rollers forming respective nips for receiving the material, the rollers being rotatable to advance the material, the second pair of rollers being located after the first pair of rollers in the direction of advance of the material and being rotatable at a faster speed than the first pair of rollers to stretch the material between the pairs of rollers and a measuring system for providing a measure of the respective lengths of the material that have passed through each pair of rollers and a measure of the torque on at least one of the rollers, and for deriving therefrom instantaneous measures of the Young's Modulus of material between the pairs of rollers.

According to a second aspect of the invention, there is provided apparatus for testing a piece of elastic material such as leather comprising first and second pairs of rollers forming respective nips for receiving the material, the rollers being rotatable to advance the material, the second pair of rollers being located after the first pair of rollers in the direction of advance of the material and being rotatable at a faster speed than the first pair of rollers to stretch the material between the pairs of rollers, a measuring system being provided for sensing the rotational positions of the rollers and the torque on the rollers and deriving therefrom instantaneous measures of the Young's Modulus of material between the pairs of rollers.

In this way, an idea can be provided of the strength of the material along the whole length or the whole breadth of the piece of the material. This can allow accurate cutting of the material to give desired qualities.

The following is a more detailed description of embodiments of the invention, by way of example, reference being made to the accompanying drawings in which:-
Figure 1 is a schematic view of a first testing apparatus for testing leather; and
Figure 2 is a schematic view of a second testing apparatus for testing leather.

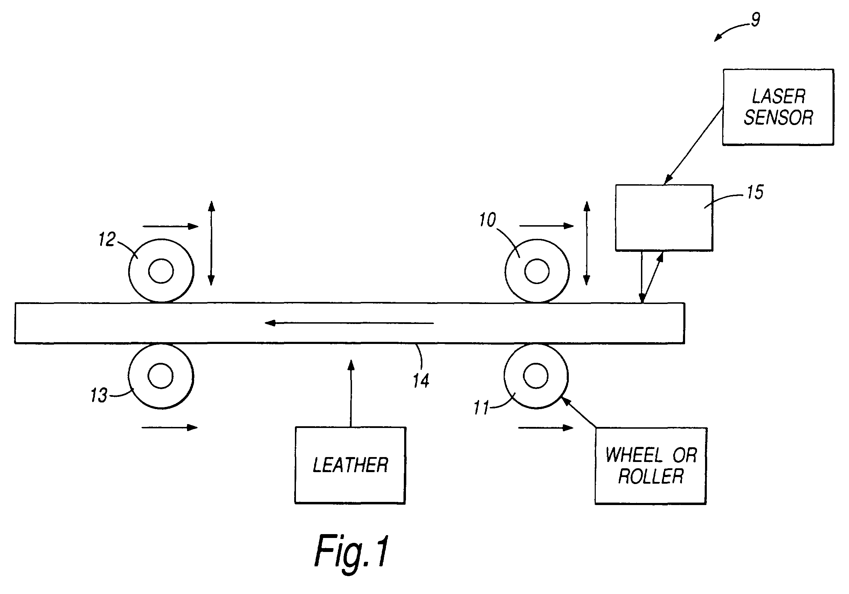

Referring to Figure 1, the first testing apparatus 9 comprises a first pair of rollers 10,11 and a second pair of rollers 12,13. Each pair of rollers, 10,11; 12,13, forms a nip between which passes a piece of leather 14 to be tested.

The distance between the first pair of rollers 10,11 and the second pair of rollers 12,13 is adjustable. In addition, the spacing between the axes of the rollers in the first pair of rollers 10,11 and the spacing between the axes of the rollers in the second pair of rollers 12,13 are also adjustable to adjust the width of the nip between each pair of rollers 10,11 or 12,13.

Each roller 10,11; 12,13, is driven by a motor and the speed and acceleration of each motor is controlled.

In addition, each roller 12,13, of the second pair of rollers has an associated torque sensor for monitoring the torque applied to the associated roller 12,13. Each roller also has an associated position sensor for sensing the rotational position of the associated roller. Further, a displacement sensor senses the distance between the axis of one roller 12 of the second pair of rollers and the axis of the other roller 13 of the second pair of rollers in a direction along a line normal to said axes and passing through said axes. A fourth sensor determines the force applied between one roller 12 of the second pair of rollers and the other roller 13 of the second pair of rollers.

In addition, as seen in the drawing, a laser sensor 15 gives the surface profile of the leather.

The testing apparatus described above with reference to the drawings can be used to test a number of characteristics of the leather 14.

First, it can be used to provide instantaneous measurements of Young's Modulus of the leather in a direction lying in the plane of the leather as the leather passes between the first and second pairs of rollers, 10,11 to determine the location of low strain regions. To do this, the leather is fed between the first and second pairs of rollers 10,11 and 12,13 and the rollers 10,11; 12,13 are all driven at the same speed. As seen in Figure 1, the second pair of rollers 12,13 are located after the first pair of rollers 10,11 in the direction of advance of the leather 19. Next, the second pair of rollers, 12,13 are rotated at a slightly higher speed than the first pair of rollers, 10,11. The effect of this is to stretch the leather 14 between the pairs of rollers.

While the second pair of rollers 12,13 are rotated faster than the first pair 10,11, the length of leather 14 that passes through the second pair of rollers 12,13 in any period is longer than the length of leather 14 that passes through the first pair of rollers 10,12 in that period, the leather 14 passing through the second pair of rollers being stretched compared to the leather passing through the first pair of rollers 10,11. The difference between the lengths of leather passing between the two pairs of rollers 10,11;12,13 is indicative of the extent to which the leather 14 is stretched.

The outputs of the torque sensors on the rollers 12,13 of the second pair of rollers and the outputs of the position sensors on all the rollers 10,11; 12,13 (which give a measure of the lengths of leather that have passed through the pairs of rollers 10,11;12,13) can be used by a measuring system (not shown) to provide an instantaneous measure of the stress and strain on the region of leather 14 extending between the first pair of rollers, 10,11 and the second pair of rollers 12,13. From this can be calculated the Young's Modulus of the region which will provide an idea of the strength of the region. Thus the testing apparatus can give a number of Young's Modulus values corresponding to such regions spaced over the leather 14.

These measurements can also be used to assess the hysteresis of the leather 14.

The stress and strain determined as described above can also be used to produce a measure of softness. Although softness is in general considered a subjective measurement, the results produced from the apparatus described above with reference to the drawing produces an acceptable indication of softness.

The apparatus can also be used to determine shear stress across the thickness of the leather 14. This is achieved by holding the first pair of rollers 10,11 stationary and rotating the rollers 12,13 of the second pair in opposite directions. The measuring system monitors the torque applied to the rollers 12,13 and from this, and the relative rotational positions of the rollers 12,13, the stress and strain in the leather 14 across its thickness can be determined.

This can help identify low strain regions.

The compressibility across the thickness of the leather 14 can be determined by closing the nip between the rollers 12,13 of the second pair of rollers, measuring the reduction in the size of the nip and also measuring the force applied by the rollers 12,13. From this, the measuring system can produce a measurement of compressibility of the leather across its thickness. It follows, from the previous test, that the apparatus can also measure the thickness by measuring the distance between the axes of the rollers 12,13 (i.e. the width of the nip) at a predetermined compression force.

As described above, the laser sensor 15 can be used to measure the surface profile or roughness of the specimen of the leather 14 while it is moving.

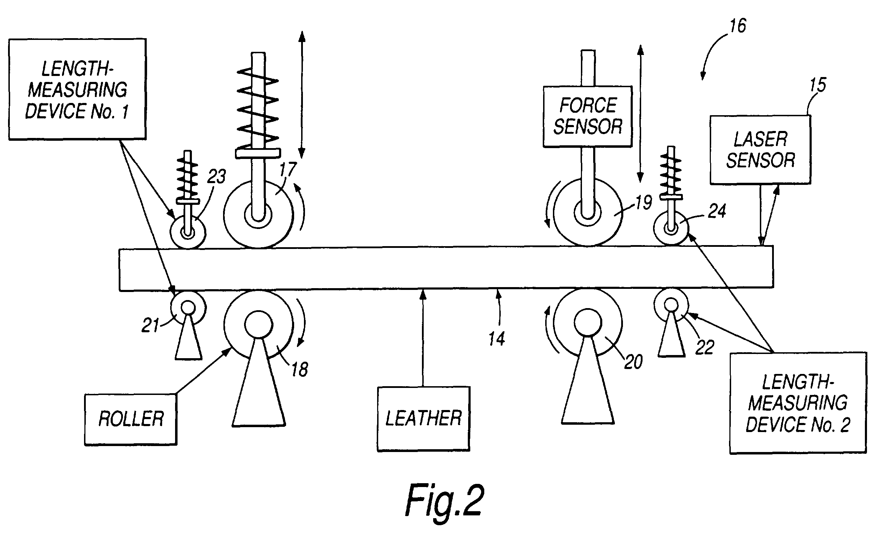

A second testing apparatus 16 is shown in Figure 2. Features of the second apparatus 16 common to the first apparatus 9 are given the same reference numerals and are not described in detail.

The second apparatus 16 has first and second pairs of rollers 17,18;19,20 that are the same as the first and second pairs of rollers 10,11;12,13 of the first apparatus 9, but which are not provided with sensors for detecting the rotational positions of the rollers 17-20.

Instead the second apparatus 16 is provided with a first measuring wheel 21 located adjacent the first pair of rollers 17,18, before the first pair in the direction of advance of the leather, and a second measuring wheel 22 located adjacent the second pair of rollers 19,20 after the second pair in the direction of advance of the leather 14. Each measuring wheel 21,11 is provided with a respective position sensor (not shown) for sensing the rotational positions of the wheels 21,22.

Additionally, the second apparatus is provided with first and second spring-loaded wheels 23,24. Each spring-loaded wheel 23,24 is located opposite a respective one of the measuring wheels 21,22 and serves to press the leather 14 into contact with the associated measuring wheel 21,22.

The second apparatus 16 is used with a measuring system (not shown) as described for the first apparatus 9.

The second apparatus 16 operates in a similar manner to the first apparatus 9. However, the measuring system uses the outputs of the position sensors on the measuring wheels 21,22 (together with the outputs of the torque sensors on the rollers 17,18;19,20) to provide instantaneous measures of the stress and strain on regions of the leather 14 extending between the pairs of rollers. Young's Modulus values are calculated from these measures as for the first apparatus 9. As the first and second measuring wheels 21,22 are located adjacent the first and second pairs of rollers 17,18;19,20 respectively, the position sensors on the measuring wheels give a measure of the lengths of leather 14 that have passed through the two pairs of rollers 17,18;19,20.

The second apparatus 16 can also be used to determine the softness, compressibility and thickness at a predetermined compression force, as described above for the first apparatus 9. Additionally, if the rollers 17,18;19,20 are provided with rotational position sensors (in addition to those provided on the measuring wheels 21,22) the second apparatus 16 can also be used to determine shear stress across the thickness of the leather 14, as described for the first apparatus 9.

It will be appreciated that the first and second testing apparatus described above need not be used to determine all the characteristics described above; they could be used to determine only any one of them. The systems need not be used with leather; they could be used with other elastic materials.

It will also be appreciated that the first and second testing apparatus described above can be used as "on-line" devices monitoring continuously a piece of leather as the leather is fed to processing machinery.

## Claims

1. Apparatus for testing a piece of elastic material such as leather comprising first and second pairs of rollers forming respective nips for receiving the material, the rollers being rotatable to advance the material, the second pair of rollers being located after the first pair of rollers in the direction of advance of the material and being rotatable at a faster speed than the first pair of rollers to stretch the material between the pairs of rollers, and a measuring system for providing a measure of the respective lengths of the material that have passed through each pair of rollers and a measure of the torque on at least one of the rollers, and for deriving therefrom instantaneous measures of the Young's Modulus of material between the pairs of rollers.

2. Apparatus according to claim 1, wherein the measure of the respective lengths comprises information about the respective rotational positions of at least one of the rollers of each pair, the measuring system sensing said rotational positions.

3. Apparatus according to claim 1, wherein the measuring system comprises a first wheel located adjacent the first pair of rollers and a second wheel located adjacent the second pair of rollers, the wheels rotating in contact with the material as the material advances, the measure of the respective lengths comprising information about the respective rotational positions of the wheels, the measuring system sensing said rotational positions.

4. Apparatus according to claim 3, wherein the first wheel is located before the first pair of rollers in the direction of advance and the second wheel is located after the second pair of rollers in the direction of advance.

5. Apparatus according to claim 3 or claim 4, wherein the material is pressed against the first and second wheels by respective spring loaded wheels, each spring located wheel being located at the opposite side of the material to the corresponding one of the first and second wheels.

6. Apparatus according to any preceding claim, wherein the measuring system derives from said measure of the respective lengths and said measure of the torque, a measure of the softness of the material.

7. Apparatus for testing a piece of elastic material such as leather comprising first and second pairs of rollers forming respective nips for receiving the material, the rollers being rotatable to advance the material, the second pair of rollers being located after the first pair of rollers in the direction of advance of the material and being rotatable at a faster speed than the first pair of rollers to stretch the material between the pairs of rollers, a measuring system being provided for sensing the rotational positions of the rollers and the torque on the rollers and deriving therefrom instantaneous measures of the Young's Modulus of material between the pairs of rollers.

8. Apparatus according to claim 7, wherein said measuring system derives from said rotational positions and said torque on the rollers, a measure of the softness of the material.

9. Apparatus according to any one of claims 2, 6 or 7, wherein, with the first and second pairs of rollers halted, one of said pairs of rollers are rotatable in respective opposite directions, the measuring system sensing the torque on the oppositely rotated rollers and the relative rotational positions of the oppositely rotated rollers and deriving therefrom a measure of the Young's Modulus of the material in a direction extending generally along a line normal to the axes of the oppositely rotated rollers and passing through the axes.

10. Apparatus according to any preceding claim, wherein the rollers of one of said pairs of rollers are operable to adjust the size of the nip between the rollers to compress the material between the rollers of said pair, the measuring system sensing the size of the nip and the compressive force and deriving therefrom the compressibility of the material.

11. Apparatus according to claim 10, wherein the measuring system derives a measurement of the thickness of the material from the size of the nip between said pair of rollers at a predetermined compressive force.

12. Apparatus according to any preceding claim and including a sensor for measuring the surface profile of the material to provide an indication of roughness.

13. Apparatus according to claim 12 wherein said sensor is a laser sensor.
